# EUROPÄISCHE PATENTSCHRIFT

(11) **EP 2 675 432 B1**
(45) Veröffentlichungstag und Bekanntmachung des Hinweises auf die Patenterteilung: **20.04.2016**
(21) Anmeldenummer: 12707218.9
(22) Anmeldetag: 16.02.2012
(51) Int. Cl.: A61K 9/00, A61K 33/04, A61P 15/02

(54) **PHARMAZEUTISCHE ZUBEREITUNG ENTHALTEND SELENIT- ODER SELENITHALTIGE VERBINDUNGEN ZUR BEHANDLUNG VON CERVIX-DYSPLASIEN ODER -KARZINOMEN**
PHARMACEUTICAL COMPOSITION COMPRISING SELENITE OR A SELENITE COMPRISING COMPOUND FOR THE TREATMENT OF CERVICAL DYSPLASIA OR CARCINOMA
COMPOSITION PHARAMCEUTIQUE CONTENTANT SÉLÉNITE OU UN COMPOSÉ DE SÉLÉNITE POUR LE TRAITEMENT DE DYSPLASIE OU CANCER CERVICAL

(30) Priorität: 16.02.2011 AT 2012011
(43) Veröffentlichungstag der Anmeldung: 25.12.2013
(73) Patentinhaber: Selo Medical GmbH, 5585 Unternberg (AT)
(72) Erfinder: FUCHS, Norbert, A-5571 Mariapfarr (AT)
(74) Vertreter: Sonn & Partner Patentanwälte
(86) Internationale Anmeldenummer: PCT/AT2012/000032
(87) Internationale Veröffentlichungsnummer: WO 2012/109685

(56) Entgegenhaltungen:
- WO-A1-03/047604
- RUDOLF E ET AL: "Selenium activates p53 and p38 pathways and induces caspase-independent cell death in cervical cancer cells", CELL BIOLOGY AND TOXICOLOGY, KLUWER ACADEMIC PUBLISHERS, DO, Bd. 24, Nr. 2, 3. Juli 2007 (2007-07-03), Seiten 123-141, XP019570904, ISSN: 1573-6822
- LIPING FU ET AL: "Proteomic study on sodium selenite-induced apoptosis of human cervical cancer HeLa cells", JOURNAL OF TRACE ELEMENTS IN MEDICINE AND BIOLOGY, Bd. 25, Nr. 3, 1. Juli 2011 (2011-07-01), Seiten 130-137, XP55023165, ISSN: 0946-672X, DOI: 10.1016/j.jtemb.2011.06.001

## Beschreibung

Die Erfindung betrifft pharmazeutische Zusammensetzungen, enthaltend selenithältige Verbindungen.

Entzündliche und/oder degenerative Veränderungen des weiblichen Gebärmutterhalses stellen ein immer größer werdendes gesundheitliches Problem dar. Vom griechischen Arzt George Papanicolaou entwickelt, werden zervikale Zellabstriche - nach der sogenannten Münchener Nomenklatur II - klassifiziert. Dabei entspricht die Klassifizierung PAP I einem Normalbefund, PAP II einer leicht entzündlichen und/oder degenerativen Veränderung, PAP III kontrollbedürftigen und nicht einschätzbaren Zellbildern, PAP IIID einer Dysplasie, PAP IV schwerwiegenden Karzinom-Vorstufen und PAP V einem malignen Tumor. Die Dysplasie-Formen PAP IIID und PAP IV werden zytologisch weiter differenziert in sogenannte "cervikale intraepitheliale Neoplasien" (CIN) der Stufen CIN 1 (leichte), CIN 2 (mäßige) und CIN 3 (schwere Dysplasie). Analog zur histologischen Klassifikation CIN 1 (PAP IIID), über CIN 2 (ebenfalls PAP IIID) zu CIN 3 (PAP IV) wird im angloamerikanischen Raum auch die sogenannte Bethesda-Klassifizierung herangezogen. Dabei entspricht die "Low-Grade Squamous Intraepithelial Lesion" (LSIL) der Münchener Klassifizierung CIN 1, wogegen höhergradige Zellveränderungen "High-Grade Squamous Intraepithelial Lesions" (HSIL) den WHO-Klassifizierungen CIN 2 und CIN 3 entsprechen.

Dabei beträgt die Rückbildungstendenz von leichten Dysplasien (PAP IIID/CIN 1/LSIL) zu einem Normalbefund (PAP I bzw. PAP II) innerhalb eines Jahres im Mittel nur knapp 15%. Die Progressionstendenz leichter Dysplasien (PAP IIID/CIN 1/LSIL) zu höhergradigen Dysplasien zeigt aktuell eine mittlere jährliche Übergangswahrscheinlichkeit von mehr als 7%, jene von höhergradigen Dysplasien zu Uteruskarzinomen 0,74%.

Aktuelle internationale gynäkologische Leitlinien zur Therapie von Cervikalen Intraepithelialen Neoplasien (CIN) und Mikrokarzinomen der Cervix uteri umfassen, je nach Lage und Schweregrad der Zellveränderungen, eine Oberflächendestruktion des betroffenen Gewebes, eine Konisation mittels Skalpells, Lasers oder LEEPs (Loop Electrosurgical Excision Procedure) oder eine Hysterektomie. Andere, nicht-chirurgische Therapien sind aktuell nicht bekannt.

Entzündliche Gewebeprozesse sind auf elektrochemischer Ebene immer von einem (regionalen) Anstieg sogenannter Reactive-Oxygene-Species (ROS), von Radikalen und Peroxiden begleitet. Der Kompetenz des körpereigenen Immunsystems und dem Körperbestand endogener und exogener Antioxidantien kommt im Rahmen der spontanen Linderung dieser oxidativen Entzündungsfaktoren eine große biologische Rolle zu. Zahlreiche wissenschaftliche Publikationen und internationale Patentschriften (u.a. WO 2001/093910 A2 und WO 2003/047604 A1) konnten mittlerweile entzündungshemmende und antivirale Effekte von antioxidativen Zusammensetzungen nachweisen.

Aufgabe der vorliegenden Erfindung war es, neue Mittel zur Vorbeugung und Behandlung von Cervix-Entzündungen, -Dysplasien und/oder -Karzinomen zur Verfügung zu stellen.

Demgemäß betrifft die vorliegende Erfindung eine pharmazeutische Zusammensetzung, enthaltend selenithältige Verbindungen und pharmazeutisch akzeptable Säuren, ausgewählt aus Zitronensäure, Essigsäure, Äpfelsäure, Kohlensäure, Schwefelsäure, Salpetersäure, Salzsäure, Fruchtsäuren (z.B. Apfelsäure, Zitronensäure, Weinsäure, Oxalsäure und Fumarsäure, insbesondere Zitronensäure) oder Mischungen davon, zur Anwendung zur Behandlung von Cervix-Entzündungen, -Dysplasien und/oder -Karzinomen, wobei die Zusammensetzung cervikaler Zellveränderungen mit einem PAP-Score ≥ PAP III und/oder einem CIN-Score ≥ CIN 1 eingesetzt wird.

Mit der vorliegenden Erfindung konnte überraschend festgestellt werden, dass sich mit einer konsequenten, lokalen Applikation antioxidativer selenhältiger Präparationen (gemäß WO 2001/093910 A2 und WO 2003/047604 A1) in vivo auch auf neg. Zellveränderungen (Dysplasien und Karzinome) im Rahmen der Cervixkarzinom-Früherkennung positiv auswirkt. Die vorliegende Erfindung eignet sich sowohl für HPV-induzierte als auch für nicht-HPV-induzierte uterine Störungen. Dies ist von großer praktischer Relevanz, da in der gynäkologischen Praxis häufig auf einen HPV-Nachweis oder gar auf eine HPV-Spezifizierung verzichtet wird, da der positive Nachweis einer HPV-Infektion oft keinen Einfluss auf die nachfolgende Therapieentscheidung hat. Die Behandlung nicht-HPV-induzierter uteriner Störungen gemäß der vorliegenden Erfindung (also die Verwendung der erfindungsgemäßen Präparate zur Behandlung von bzw. als Medikamente für Cervix-Entzündungen, -Dysplasien und/oder -Karzinomen) stellt aber eine ganz besonders bevorzugte Ausführungsform dar. Daraus ergibt sich auch, dass die vorliegende Erfindung eben keine gegen einen bestimmten Krankheitserreger gerichtete Strategie darstellt, sondern eine gezielt auf die Cervix-Entzündungen,-Dysplasien und/oder -Karzinome abzielende Behandlung ist, also auch eingesetzt werden kann, (lange) nachdem ein möglicher Krankheitserreger die Krankheitssymptome ausgelöst hat.

Es hat sich gezeigt, dass durch Versetzen von wässerigen Lösungen anorganischer Selen-Verbindungen mit derartigen Säuren eine Zusammensetzung mit erhöhtem antioxidativen Potential zur Verfügung gestellt werden kann. Die erfindungsgemäß hergestellten Zusammensetzungen, also insbesondere Lösungen, Gele, Emulsionen, Suspensionen, Salben usw., zeigen dabei die erfindungsgemäßen therapeutischen Effekte, weil diese - durch die Anwesenheit der Säuren - erfindungsgemäß in einer Weise angewendet werden können, in der dieses erhöhte antioxidative Potential zumindest zeitweise erhalten bleibt. Dies ist dann der Fall, wenn bei der Anwendung am therapeutischen Zielort das gesteigerte antioxidative Potential noch vorhanden ist und nicht beispielsweise durch Applikationslösungen oder Körpersäfte wie Blut (z.B. bei intravenöser Anwendung) oder Magen oder Darminhalte (bei oraler Anwendung) verdünnt worden ist.

Demgemäß betrifft die vorliegende Erfindung vorzugsweise die topische, mukosale bzw. intravaginale Verabreichung dieser Präparate für die externe Anwendung (also topisch oder bukkal) bzw. für die direkte Applikation an Schleimhäute (mukosale Anwendung). Typische Verabreichungsformulierungen, die zur topischen, mukosalen bzw. intravaginalen Verabreichung geeignet sind, sind einem Fachmann bekannt und in den einschlägigen Arzneimittelbüchern vorbeschrieben.

Die erfindungsgemäße Zusammensetzung kann neben den wesentlichen Inhaltsstoffen Selenit und den Säuren auch weitere geeignete Inhaltsstoffe und/oder pharmazeutisch geeignete Exzipienten enthalten. Dabei enthält die erfindungsgemäße Zusammensetzung Selenit vorzugsweise in einer Menge von 1 bis 500mg, noch bevorzugter 10 bis 100mg, insbesondere 30 bis 70mg, pro 100g Zusammensetzung. Vorzugsweise enthält die erfindungsgemäße Zusammensetzung Selenit in Form von Natrium-Selenit (liegt meist als Pentahydrat vor; ab 40°C gibt das Pentahydrat das Kristallwasser ab).

Unabhängig davon enthält die erfindungsgemäße Zusammensetzung vorzugsweise eine (oder mehrere) Säure(n) in einer Gesamtmenge von 1mg bis 10g Säure, noch bevorzugter 10mg bis 5g Säure, insbesondere 100mg bis 1g Säure, pro 100g Zusammensetzung (insbesondere, wenn die Säure in fester Form zugegeben wird. Alternativ dazu kann die Säure auch in flüssiger Form (z.B. mit Wasser, also als wässrige Lösung) zugegeben werden. Wasser bzw. wässrige Lösungen, gegebenenfalls mit weiteren Inhaltsstoffen, können der erfindungsgemäßen Zusammensetzung in einer Menge von 0 bis (etwa) 99,9g, vorzugsweise 50 bis 99g, insbesondere 80 bis 98g, pro 100g Zusammensetzung zugesetzt sein.

Gemäß einer bevorzugten Ausführungsform wird die vorliegende Erfindung als Gel zur Verfügung gestellt. Demgemäß enthält die erfindungsgemäße Zusammensetzung vorzugsweise einen Gelbildner. Als Gelbildner können anorganische sowie organische Hydrogelbildner eingesetzt werden. Besonders geeignete Gelbildner sind Cellulosederivate, insbesondere Carboxymethylcellulose, Methylcellulose, Hydroxypropylcellulose und besonders Hydroxyethylcellulose. Bevorzugter Weise werden die Gelbildner, insbesondere Hydroxyethylcellulose, in einer Gesamtkonzentration von 0,1 g bis 30 g, noch bevorzugter 0,5 g bis 5 g, insbesondere 1 g bis 3 g, pro 100 g Zusammensetzung, eingesetzt.

Eine besonders bevorzugte Variante der erfindungsgemäßen Gel-Zusammensetzung enthält Siliziumdioxid, insbesondere hochdisperses Siliziumdioxid, z.B. gemäß der WO 2001/85852 A1, als technologischen Suspensionsträger und/oder als Adsorbens. Bevorzugt werden dabei 100mg bis 50g, noch bevorzugter 500mg bis 10g, insbesondere 1g bis 5g, SiO₂ pro 100g Zusammensetzung eingesetzt.

Die erfindungsgemäße Zusammensetzung hat bevorzugter Weise einen pH-Wert von unter 7,0, noch bevorzugter unter 5,0, insbesondere von 4,0 bis 2,5.

Die erfindungsgemäße Zusammensetzung liegt vorzugsweise als Lösung, Emulsion, Salbe oder Schwamm (Tampon) vor. Dabei kann sie günstiger Weise weitere Hilfsstoffe und/oder weitere wirksame Komponenten enthalten, insbesondere Puffersubstanzen, Farbstoffe, Stabilisierungsstoffe, Konservierungsmittel, Trägersubstanzen oder Kombinationen davon. Bevorzugte Beispiele derartiger Substanzen sind Maltodextrin, Aromastoffe, wie z.B. Zitronenaroma, Pfefferminzöl, Kaliumsorbat und Natriumbenzoat (als Konservierungsmittel), etc..

Bevorzugte weitere Wirksubstanzen sind dabei Antibiotika, antivirale Mittel, Antimykotika, schmerzhemmende Mittel, entzündungshemmende Mittel oder Kombinationen davon.

Die erfindungsgemäße Zusammensetzung hat sich überraschender Weise als besonders wirksam zur Behandlung cervikaler Zellveränderungen mit einem PAP-Score ≥ PAP III und/oder einem CIN-Score ≥ CIN 1 erwiesen. Insbesondere kann die vorliegende Erfindung zur Behandlung cervikaler Entzündungen mit einem PAP-Score von PAP III und PAP IIID eingesetzt werden.

Besonders hervorzuheben ist auch, dass sich die erfindungsgemäße Zusammensetzung zur Behandlung von Cervix-Karzinomen eignet.

Die vorliegende Erfindung betrifft gemäß einem weiteren Aspekt ein Verfahren zur Behandlung von Cervix-Entzündungen, -Dysplasien und/oder -Karzinomen, bei welchem die erfindungsgemäßen Zusammensetzungen in einer effektiven Menge an Patientinnen verabreicht wird, die an diesen Erkrankungen leiden. Bevorzugte Dosierungen können (z.B. bei einem Gel) von 0,005 bis 0,1 g Natriumselenit-Pentahydrat pro 100 g Gel betragen, insbesondere von 0,01 bis 0,1 g pro 100g Gel.

Die Erfindung wird anhand der nachfolgenden Beispiele, auf die sie selbstverständlich nicht eingeschränkt ist, näher erläutert:

### Beispiel 1: Erstellung eines angesäuerten Natriumselenit-Gels

Ein angesäuertes Natriumselenit-Gel wurde mit folgender Zusammensetzung hergestellt (pro 100 g):

| | |
|---|---|
| Natriumselenit-Pentahydrat | 0,050 g |
| Siliziumoxid, hochdispers | 0,200 g |
| Zitronensäure | 0,496 g |
| Natriumbenzoat | 0,050 g |
| Kaliumsorbat | 0,099 g |
| Hydroxyethylcellulose | 1,985 g |
| Wasser | 97,120 g |
| | 100,000 g |

### Beispiel 2: Behandlung von Cervix-Dysplasien

Design: Mulitzentrische Pilotstudie
Inklusionskriterien:
   Alter > 19 Jahre
   PAP ≥ III < IV

### Durchführung:

Intravaginale Anwendung von 5 ml Natriumselenit-Gel 1 x pro Tag nach Vorliegen der Diagnose PAP ≥ III < IV über einen Zeitraum von 90 Tagen. Während der Menses ist die Anwendung auszusetzen. Kontrolluntersuchung nach 90 Tagen Gelanwendung.

### Ergebnisse:

Von 31 Patientinnen hatten 27 (87,1%) eine Response; 4 Patientinnen (12,9%) waren Non-Responder.

### Beispiel 3: Wirkung des angesäuerten Natriumselenit-Gels

| Initialen | Alter | erste Visite | letzte Visite | PAP Beginn | PAP Ende | HPV Beginn | HPV Ende |
|---|---|---|---|---|---|---|---|
| CC | 45 | 15.07.2010 | 05.10.2010 | III D | II | neg. | n.b. |
| AU | 47 | 20.07.2010 | 12.10.2010 | III D | II | n.b. | n.b. |
| IG | 28 | 20.07.2010 | 09.09.2010 | IV | IV | pos. | n.b. |
| NK | 34 | 03.08.2010 | 03.11.2010 | III D | III | neg. | n.b. |
| KS | 44 | 03.08.2010 | 03.11.2010 | III | III | neg. | n.b. |
| RS | 19 | 21.07.2010 | 28.10.2010 | III D | III D | pos. | pos. |
| BK | 49 | 27.07.2010 | 27.10.2010 | III D | II | pos. | n.b. |
| MJ | 42 | 27.07.2010 | 27.10.2010 | III D | II | n.b. | n.b. |
| AU | 19 | 14.04.2010 | 03.11.2010 | IIID | II | pos. | n.b. |
| TF | 25 | 28.07.2010 | 28.10.2010 | III D | II | pos. | neg. |
| RH | 49 | 28.07.2010 | 28.10.2010 | III D | II | n.b. | n.b. |
| SH | 32 | 29.07.2010 | 02.11.2010 | III D | II | pos. | n.b. |
| AK | 27 | 02.08.2010 | 03.11.2010 | III D | II | pos. | n.b. |
| SL | 27 | 29.07.2010 | 29.10.2010 | III | II | pos. | n.b. |
| AG | 71 | 02.08.2010 | 04.11.2010 | III | II | n.b. | pos. |
| ER | 28 | 26.08.2010 | 02.11.2010 | III D | II | pos. | pos. |
| BS | 53 | 10.05.2010 | 30.09.2010 | III D | III D | pos. | n.b. |
| RS | 52 | 23.06.2010 | 11.11.2010 | III | III | neg. | neg. |
| DG | 48 | 18.05.2010 | 02.11.2010 | III D | II | n.b. | n.b. |
| MJ | 19 | 10.05.2010 | 09.08.2010 | III D | II | pos. | neg. |
| RM | 62 | 27.05.2010 | 06.10.2010 | III | II | n.b. | n.b. |
| AF | 38 | 22.06.2010 | 16.08.2010 | III D | II | pos. | n.v. |
| MF | 49 | 28.07.2010 | 30.06.2010 | III D | II | pos. | pos. |
| PK | 56 | 26.05.2010 | 02.11.2010 | III D | II | neg. | n.b. |
| IR | 41 | 08.06.2010 | 17.08.2010 | III | IV | n.b. | n.b. |
| BP | 56 | 14.06.2010 | 03.11.2010 | III D | II | pos. | n.v. |
| EN | 47 | 02.06.2010 | 11.11.2010 | III D | II | pos. | n.v. |
| IP | 40 | 26.07.2010 | 26.08.2010 | III D | II | pos. | n.v. |
| MW | 47 | 26.07.2010 | 26.08.2010 | III D | n.b. | n.b. | n.v. |
| MR | 50 | 02.08.2010 | 02.09.2010 | III D | IV | pos. | n.v. |
| DB | 22 | 08.07.2010 | 11.11.2010 | III | II | pos. | n.v. |
| YT | 32 | 19.08.2010 | 11.11.2010 | III | II | n.b. | n.v. |
| KP | 67 | 10.08.2010 | 03.11.2010 | III | II | neg. | n.b. |
| RK | 45 | 24.06.2010 | 20.09.2010 | III | II | neg. | neg. |
| CS | 50 | 30.08.2010 | 08.11.2010 | III | III | n.b. | n.v. |
| ML | 28 | 03.08.2010 | 03.11.2010 | III | II | neg. | |
| FP | 43 | 10.08.2010 | 11.11.2010 | III D | II | pos. | n.b. |
| CD | 44 | 07.09.2010 | 14.12.2010 | III D | II | pos. | n.b. |
| KW | 21 | 12.08.2010 | 30.11.2010 | III D | III D | pos. | n.b. |
| EL | 52 | 13.08.2010 | 18.11.2010 | III | II | n.b. | n.b. |
| ES | 38 | 10.08.2010 | 21.09.2010 | III | II | n.b. | n.b. |
| GT | 56 | 14.09.2010 | 14.12.2010 | III D | II | n.b. | n.b. |
| BM | 40 | 20.08.2010 | 10.11.2010 | III D | II | n.b. | n.b. |
| MP | 47 | 30.07.2010 | 16.11.2010 | III D | III D | pos. | pos. |
| GS | 38 | 19.08.2010 | 14.12.2010 | III D | II | n.b. | n.b. |

| | | | | | | | |
|---|---|---|---|---|---|---|---|
| n.b.: nicht bestimmt n.v.: nicht verfügbar neg.: negativ Pos.: positiv | | | | | | | |

Die Anwendung des angesäuerten Natriumselenit-Gels an zwei Patientinnen konnte zeigen, dass das Gel erfindungsgemäße Wirkung zeigt und effizient zur Behandlung von Cervix-Dysplasien eingesetzt werden kann.

### Beispiel 4: Behandlung eines Plattenepithelkarzinoms der Cervix uteri einer 38-jährigen Patientin mit angesäuertem Natriumselenit-Gel (hergestellt nach Beispiel 1)

Aufgrund einer ausgeprägten Gebärmutterdysplasie (Stadium PAP IV, bioptisch nach CIN 3) wurden an IG, geboren am 31.12.1975, während ihres Krankenhausaufenthaltes vom 29.5.2008 bis 2.6.2008 in einer allgemein öffentlichen österreichischen Klinik eine Konisation sowie eine Cervixcurettage durchgeführt. Weitere Untersuchungen sowie eine am entnommenen Gewebe durchgeführte histologische Untersuchung ergaben den Befund eines invasiven Plattenepithelkarzinoms der Cervix uteri sowie eines Karzinoms in situ mit Stadium FIGU IB 1. Ein anschließender stationärer Aufenthalt der Patientin in der gynäkologischen Abteilung einer weiteren österreichischen Klinik bestätigte diesen Befund.

CT-, MRI- und sonographische Untersuchungen ergaben einen offensichtlich malignen Tumor mit einer Ausdehnung von 10 - 12 mm mit Sitz im kranialen Bereich der Cervix uteri, übergreifend auf den Isthmus uteri, allerdings noch ohne lymphogene Metastasierung. Diese Befunde führten - nicht zuletzt auch aufgrund des Sitzes des Resttumors im Bereich des Isthmus uteri - zur Therapieempfehlung einer Radikaloperation nach Wertheim PIVER II, da eine Uterus-erhaltende Operation aufgrund der Lage des Tumors nicht möglich war. Trotz des enormen Zeitdruckes eine Entscheidung zu treffen, holte die Patientin eine medizinische Zweitmeinung ein und entschloss sich zu einer möglicherweise Uteruserhaltenden Therapie mit dem erfindungsgemäßen Präparat. Eine zeitlich engmaschige gynäkologische, radiologische und histologische Begleitung der Patientin ergab nach viermonatiger Therapie mit dem erfindungsgemäßen Gel eine deutliche Verkleinerung des Tumors sowie ein Abflammen der Entzündung. Nach weiteren zwei Monaten Therapie mit dem erfindungsgemäßen Präparat war der Tumor verschwunden, der histologische Abstrich ergab einen Rückgang der Entzündung auf PAP II+.

## Patentansprüche

1. Pharmazeutische Zusammensetzung, enthaltend selenithältige Verbindungen und pharmazeutisch akzeptable Säuren, ausgewählt aus Zitronensäure, Essigsäure, Äpfelsäure, Kohlensäure, Schwefelsäure, Salpetersäure, Salzsäure, Fruchtsäuren oder Mischungen davon, zur Anwendung zur Behandlung cervikaler Zellveränderungen mit einem PAP-Score ≥ PAP III und/oder einem CIN-Score ≥ CIN 1.

2. Zusammensetzung zur Anwendung nach Anspruch 1, **dadurch gekennzeichnet, dass** sie zur topischen, mukosalen bzw. intravaginalen Verabreichung hergerichtet ist.

3. Zusammensetzung zur Anwendung nach Anspruch 1 oder 2, **dadurch gekennzeichnet, dass** sie einen Gelbildner enthält

4. Zusammensetzung zur Anwendung nach einem der Ansprüche 1 bis 3, **dadurch gekennzeichnet, dass** sie einen Hydrogelbildner enthält, insbesondere Cellulosederivate.

5. Zusammensetzung zur Anwendung nach einem der Ansprüche 1 bis 4, **dadurch gekennzeichnet, dass** sie einen Gelbildner, ausgewählt aus Carboxymethylcellulose, Hydroxypropylcellulose, Methylcellulose und Hydroxyethylcellulose, insbesondere Hydroxyethylcellulose, enthält.

6. Zusammensetzung zur Anwendung nach einem der Ansprüche 1 bis 5, **dadurch gekennzeichnet, dass** sie als Gel vorliegt und hochdisperses Siliziumdioxid als technologischen Suspensionsträger und/oder als Adsorbens enthält.

7. Zusammensetzung zur Anwendung nach einem der Ansprüche 1 bis 6, **dadurch gekennzeichnet, dass** sie einen pH-Wert von unter 7,0, vorzugsweise unter 5,0, insbesondere von 4,0 bis 2,5 aufweist.

8. Zusammensetzung zur Anwendung nach einem der Ansprüche 1 bis 7, **dadurch gekennzeichnet, dass** sie als Lösung, Emulsion, Salbe oder Schwamm (Tampon) vorliegt.

9. Zusammensetzung zur Anwendung nach einem der Ansprüche 1 bis 8, **dadurch gekennzeichnet, dass** sie weitere Hilfsstoffe und/oder weitere wirksame Komponenten enthält, insbesondere Puffersubstanzen, Farbstoffe, Stabilisierungsstoffe, Trägersubstanzen oder Kombinationen davon.

10. Zusammensetzung zur Anwendung nach einem der Ansprüche 1 bis 9, **dadurch gekennzeichnet, dass** sie Antibiotika, antivirale Mittel, Antimykotika, schmerzhemmende Mittel, entzündungshemmende Mittel oder Kombinationen davon enthält.

11. Zusammensetzung zur Anwendung nach einem der Ansprüche 1 bis 10, **dadurch gekennzeichnet, dass** sie zur Behandlung cervikaler Entzündungen mit einem PAP-Score von PAP III und PAP IIID eingesetzt wird.

12. Zusammensetzung zur Anwendung nach einem der Ansprüche 1 bis 11, **dadurch gekennzeichnet, dass** sie zur Behandlung von Cervix-Karzinomen eingesetzt wird.

## Claims

1. Pharmaceutical composition containing selenite-containing compounds and pharmaceutically acceptable acids, selected from citric acid, acetic acid, malic acid, carbonic acid, sulfuric acid, nitric acid, hydrochloric acid, fruit acids or mixtures thereof, for the use in the treatment of cervical cell alterations with a PAP score of ≥ PAP III and/or a CIN score of ≥ CIN 1.

2. Composition for use according to claim 1, **characterized in that** it is prepared for topical, mucosal or intravaginal administration.

3. Composition for use according to claim 1 or 2, **characterized in that** it contains a gelling agent.

4. Composition for use according to any one of claims 1 to 3, **characterized in that** it contains an aqueous gelling agent, in particular cellulose derivatives.

5. Composition for use according to any one of claims 1 to 4, **characterized in that** it contains a gelling agent selected from carboxymethylcellulose, hydroxypropylcellulose, methylcellulose and hydroxyethylcellulose, in particular hydroxyethylcellulose.

6. Composition for use according to any one of claims 1 to 5, **characterized in that** it is present in the form of a gel and contains highly dispersed silicon dioxide as a technological suspension medium and/or as an adsorbent.

7. Composition for use according to any one of claims 1 to 6, **characterized in that** it has a pH-value of less than 7.0, preferably less than 5.0 and in particular between 4.0 and 2.5.

8. Composition for use according to any one of claims 1 to 7, **characterized in that** it is present in the form of a solution, emulsion, ointment or sponge (tampon).

9. Composition for use according to any one of claims 1 to 8, **characterized in that** it contains further excipients and/or further active ingredients, in particular buffer substances, coloring agents, stabilizers, carrier substances or combinations thereof.

10. Composition for use according to any one of claims 1 to 9, **characterized in that** in contains antibiotics, antiviral agents, antimycotics, pain inhibitors, anti-inflammatory agents or combinations thereof.

11. Composition for use according to any one of claims 1 to 10, **characterized in that** it is used in the treatment of cervical inflammations with a PAP score of PAP III and PAP IIID.

12. Composition for use according to any one of claims 1 to 11, **characterized in that** it is used for the treatment of cervical carcinomas.

## Revendications

1. Composition pharmaceutique contenant des composés contenant un sélénite et des acides pharmaceutiquement acceptables choisis parmi l'acide citrique, l'acide acétique, l'acide malique, l'acide carbonique, l'acide sulfurique, l'acide nitrique, l'acide chlorhydrique, les acides de fruits ou les mélanges de ceux-ci, pour une utilisation pour le traitement de lésions des cellules cervicales présentant un score PAP ≥ PAP III et/ou un score CIN ≥ CIN 1.

2. Composition pour l'utilisation selon la revendication 1, **caractérisée en ce qu'**elle est conçue pour une administration topique, muqueuse ou intravaginale.

3. Composition pour l'utilisation selon la revendication 1 ou 2, **caractérisée en ce qu'**elle contient un gélifiant.

4. Composition pour l'utilisation selon l'une des revendications 1 à 3, **caractérisée en ce qu'**elle contient un générateur d'hydrogel, en particulier des dérivés de la cellulose.

5. Composition pour l'utilisation selon l'une des revendications 1 à 4, **caractérisée en ce qu'**elle contient un gélifiant choisi parmi la carboxyméthylcellulose, l'hydroxypropylcellulose, la méthylcellulose et l'hydroxyéthylcellulose, en particulier l'hydroxyéthylcellulose.

6. Composition pour l'utilisation selon l'une des revendications 1 à 5, **caractérisée en ce qu'**elle se présente sous forme d'un gel et contient du dioxyde de silicium hautement dispersé en tant que support de suspension technologique et/ou en tant qu'adsorbant.

7. Composition pour l'utilisation selon l'une des revendications 1 à 6, **caractérisée en ce qu'**elle présente un pH inférieur à 7,0, de préférence inférieur à 5,0, en particulier de 4,0 à 2,5.

8. Composition pour l'utilisation selon l'une des revendications 1 à 7, **caractérisée en ce qu'**elle se présente sous forme d'une solution, d'une émulsion, d'une pommade ou d'une éponge (tampon).

9. Composition pour l'utilisation selon l'une des revendications 1 à 8, **caractérisée en ce qu'**elle contient des adjuvants supplémentaires et/ou des composants actifs supplémentaires, en particulier des substances tampons, des colorants, des stabilisants, des substances supports ou des combinaisons de ceux-ci.

10. Composition pour l'utilisation selon l'une des revendications 1 à 9, **caractérisée en ce qu'**elle contient des antibiotiques, des agents antiviraux, des antimycotiques, des agents analgésiques, des agents anti-inflammatoires ou des combinaisons de ceux-ci.

11. Composition pour l'utilisation selon l'une des revendications 1 à 10, **caractérisée en ce qu'**elle est utilisée pour le traitement d'inflammations cervicales présentant un score PAP de PAP III et PAP IIID.

12. Composition pour l'utilisation selon l'une des revendications 1 à 11, **caractérisée en ce qu'**elle est utilisée pour le traitement de cancers du col de l'utérus.
